# NEUE EUROPÄISCHE PATENTSCHRIFT

(11) **EP 1 371 350 B2**
(45) Veröffentlichungstag und Bekanntmachung des Hinweises auf die Entscheidung über den Einspruch: **02.01.2013**
(45) Hinweis auf die Patenterteilung: 24.12.2008
(21) Anmeldenummer: 03090178.9
(22) Anmeldetag: 13.06.2003
(51) Int. Cl.: A61K 8/81, A61K 8/891, A61K 8/04, A61Q 19/00, A61K 8/365, A61K 8/37, A61K 8/02

(54) **Kosmetisches Gelprodukt auf Basis von Ölen und Gelierungsmitteln**
Cosmetic gel product on the basis of oils and gelling agents
Produit cosmétique sous forme de gel à la base de l'huiles et d'agents gélifiants

(30) Priorität: 14.06.2002 DE 10227409
(43) Veröffentlichungstag der Anmeldung: 17.12.2003
(73) Patentinhaber: Coty B.V., 2031 CC Haarlem (NL)
(72) Erfinder: Mateu, Juan R., Oak Ridge, NJ 07438 (US); Cheng, Guang Yu, Neshanic Sta., NJ 08853 (US); Cernasov, Domnica, Ringwood NJ 07456 (US); Macchio, Ralph, Sparta, NJ 07971 (US)
(74) Vertreter: Ziebig, Marlene

(56) Entgegenhaltungen:
- WO-A-00/26285
- WO-A-02/17870
- WO-A-93/23008
- WO-A-95/30405
- WO-A-98/27953
- WO-A1-00/21499
- JP-A- 2001 354 543
- US-A- 5 650 144
- US-A- 6 139 824
- US-B1- 6 375 938
- US-B1- 6 403 070
- "Abil EM 90 Datenblatt"

## Beschreibung

Die Erfindung betrifft ein neues kosmetisches Gelprodukt, das Öle oder Fette und gelbildende Polymere dafür enthält und das verbesserte Stabilitäts- und Struktureigenschaften hat.

Aus der WO 00/26285 sind gelierte Ester, Öle und Fette bekannt, die ein oder mehrere Diblock-Copolymere, Triblock-Copolymere, Sternpolymere, Radialpolymere als Gelierungsmittel für die Fette, Öle und/oder Ester enthalten. Die erhaltenen Gele sind dicke Flüssigkeiten, weiche Gele bis halbfeste Gele und enthalten im Falle von Diblock-Copolymeren etwa 0,1 bis 10 Gew-% und bei Triblock-Copolymeren etwa 0,1-40 Gew-% dieser Gelierungsmittel. Gemische von Diblock- und Triblock-Copolymeren, die zusammen mit Estern als Ester-Gele in den Handel kommen, enthalten etwa 75-98 Gew-% Ester und 2-25 Gew-% polymeres Geliermittel. Kohlenwasserstoff-Gele enthalten 80-99 Gew-% Kohlenwasserstoffe und 1-20 Gew-% polymeres Geliermittel.

Aus der US-A-5558872 sind mit Mineralöl vermischte Di- und Triblock-Copolymere (Geahlene^{®}) bekannt, die mit einem Fettsäuremodifikator, wie einem Fettsäureester wie Isopropylmyristate, und einem Siliconöl zu Hautschutzmitteln für Inkontinenz verarbeitet werden. Die US-A-5888492 beschreibt eine Hautkonditionier-Zusammensetzung, bestehend aus Di- und Triblock-Copolymeren, einem Mineralöl, einem Fettsäureester und nichtionischen oberflächenaktiven Mitteln.

Die WO95/30405 betrifft einen festen Antitranspirant-Stift, der zwei verschiedene Gelierungsmittel, ein Chelatisierungsmittel und ein Basisöl enthält sowie kein Wasser. Die US 6,375,938 betrifft ein Antitranspirant und Deodorant mit einem flüchtigen Silicon und einem darin gelösten Polyethylenpolymeren. Das Produkt enthält keine oberflächenaktiven Mittel und kein Wasser.

Aus der WO02/17870 ist ein Antitranspirant und Deodorant bekannt, bestehend aus einem siliconisierten Polyamid, einer Siliconflüssigkeit, einem organischen Erweichungsmittel und einer gegebenenfalls wasserhaltigen inneren Phase.

Die WO98/27953 offenbar einen Antitranspirant-Stift, der neben dem Antitranspiranz ein Gelierungsmittel und einen wasserfreien Träger enthält.

Der Erfindung liegt die Aufgabe zugrunde, ein neues kosmetisches Gelprodukt zu entwickeln und auf Basis bekannter Öle und Gelierungsmittel die Stabilitätseigenschaften des Gels zu verbessern und außerdem deutliche Strukturverbesserungen zu erreichen.

Nach einer Ausführungsform der Erfindung ist das Produkt ein fester Wasser-Gel-Stift gemäß Anspruch 1, enthaltend (in Gew-%)

| | |
|---|---|
| 12-Hydroxy Stearic Acid | 4 |
| Polybutene | 19 |
| Polyethylene (Mw 450-1000) | 8 |
| Glyceryl Stearate | 2 |
| Konservierungsmittel | 0,6 |
| Abil® EM 90 Polysiloxane (Cetyl PEG/PEG-10/1 Dimethicone) | 2 |
| Polydecene (200-800 D) | 10 |
| Phenyl Trimethicone | 7 |
| Diisostearyl Maleate | 8 |
| Farbstoffe | 7 |
| Füllstoffe | 2,4 |
| Wasser | ad 100 |
| Diisopropyl Dimer Dilinoleate | 10 |

Die erfindungsgemäßen kosmetischen Gelprodukte lasen sich nicht nur zu weichen und halbfesten Gelen verarbeiten, wie dies mit den bekannten Copolymeren für den Fachmann zu erwarten war, sondern führen zusammen mit den zusätzlichen Gelbildnern zu sehr festen und stabilen Produkten mit hoher Schmelz - und Ausblutungsbeständigkeit.

Auch die Herstellung von Grundierungen (foundations) ist auf diese Weise möglich.

Der gebildete Hautfilm zeigt eine sehr weiche Zeichnung und eignet sich daher auch zu faltenüberdeckenden, optisch schattierenden Anwendungsformen der erfindungsgemäßen Gelprodukte.

Die Verwendung der erfindungsgemäßen kosmetischen Zusammensetzungen kann zum Beispiel realisiert werden in Form von Sonnenschutzgelen, After-sun-Produkten, Tagescremes, Nachtcremes, Körpergelen, Augenkosmetik, Tiefenbehandlungsmitteln für Haare, Duschgelen, sowie in Form von dekorativer Kosmetik wie Makeup, Lippenstiften, Mascara usw. Die Herstellung dieser Produkte ist dem Fachmann auf diesem Gebiet bekannt. Bevorzugt sind frei stehende transparente oder transluzente Stifte für alle in Frage kommenden Anwendungsformen.

Die Erfindung soll nachstehend durch Beispiele näher erläutert werden. Alle Angaben erfolgen in Gewichtsprozent, sofern nichts anderes angegeben ist. Mw = Molekulargewicht.

### Beispiel 1 Wasserfreier Stift I (Referenzbeispiel)

| | |
|---|---|
| 12-Hydroxy Stearic Acid | 7 |
| Polyethylene (Mw 450-2000) | 8 |
| Konservierungsmittel | 0,3 |
| Abil EM 90 Polysiloxane | 4 |
| Polydecene (200-800 D)(New Phase Nexbase 2004fg, 2006fg und 2008 fg | 5 |
| Isopropyl Palmitate | 12 |
| Farbstoffe | 10 |
| Füllstoffe | 5 |
| Phenyl Trimethicone | 4 |
| Polybutene | ad 100 |
| Diisopropyl Adipate | 12 |

Die ölphase wird auf 90 °C erhitzt währen gerührt wird. Danach wird die ölphase auf 75 °C abgekühlt, und es werden Farbstoffe, Konservierungsmittel und Aktivstoffe hinzugegeben, wobei mit 800-1500 U/min gerührt wurde. Der Stift wird bei 70 °C gegossen.

### Beispiel 2 Wasser-Gelstift

| | |
|---|---|
| 12-Hydroxy Stearic Acid | 4 |
| Polybutene | 19 |
| Polyethylene (Mw 450-1000) | 8 |
| Glyceryl Stearate | 2 |
| Konservierungsmittel 0.6 | |
| Abil EM 90 Polysiloxane | 2 |
| Polydecene (200-800 D) | 10 |
| Phenyl Trimethicone | 7 |
| Diisostearyl Maleate | 8 |
| Farbstoffe | 7 |
| Füllstoffe | 2,4 |
| Wasser | ad 100 |
| Diisopropyl Dimer Dilinoleate | 10 |

Das Wasser wird auf 75 °C erhitzt und dann zu der separat hergestellten ölphase zugesetzt. Weiter siehe Beispiel 1.

### Beispiel 3 Wasserfreier Stift II (Referenzbeispiel)

| | |
|---|---|
| 12-Hydroxy stearic acid | 5,5 |
| Polydecene | 30 |
| Polybutene | 20,7 |
| Konservierungsmittel | 0,3 |
| Farbstoffe | 1 |
| Diisostearyl Dimer Dilinoleate | 18 |
| Isostearyl Isostearate | 23 |

Die Herstellung erfolgt gemäß Beispiel 2.

### Beispiel 4 und 5

Es wurden Stabilitätstests durchgeführt, bei denen 20 Probekörper in Stiftform bei 50 °C und einer relativen Luftfeuchtigkeit von 55 % in einem klimatisierten Raum gelagert wurden. Die Probekörper hatten eine Zusammensetzung gemäß Beispiel 2 (Beispiel 4) und gemäß Beispiel 1 (Beispiel 5).

Kontrolluntersuchungen wurden im Abstand von 2 Wochen durchgeführt. Nach 8 Wochen zeigten weder die Stifte gemäß Beispiel 4 noch die gemäß Beispiel 5 einen Schwitzeffekt. Beide Arten Stifte zeigten auch bei allen Probekörpern keinerlei Ausblühungen.

### Beispiel 6

Mit Probanden durchgeführte Tests, bei denen 15 verschiedene Bewertungen im Verlaufe der Testphase abzugeben waren, ergaben für die Zusammensetzungen gemäß Beispiel 4 folgende Gesamtbewertung (in % der Probanden):

| | |
|---|---|
| Nahezu gut (fair) | :18 % |
| gut | :12 % |
| sehr gut | :24 % |
| ausgezeichnet | :44 % |

l

## Patentansprüche

1. Fester Wasser-Gel-Stift, bestehend aus: (in Gew-%)
| | |
|---|---|
| 12-Hydroxy Stearic Acid | 4 |
| Polybutene | 19 |
| Polyethylene (Mw 450-1000) | 8 |
| Glyceryl Stearate | 2 |
| Konservierungsmittel | 0.6 |
| Cetyl PEG/PPG-10/1 Dimethicone | 2 |
| Polydecene (200-800 D) | 10 |
| Phenyl Trimethicone | 7 |
| Diisostearyl Maleate | 8 |
| Farbstoffe | 7 |
| Füllstoffe | 2,4 |
| Wasser | ad 100 |
| Diisopropyl Dimer Dilinoleate | 10 |

## Claims

1. Solid water-gel-stick consisting of (in % by weight)
| | |
|---|---|
| 12-Hydroxy stearic Acid | 4 |
| Polybutene | 19 |
| Polyethylene (Mw 450-1000) | 8 |
| Glyceryl Stearate | 2 |
| Preservative | 0.6 |
| Cetyl PEG/PPG-10/1 Dimethicone | 2 |
| Polydecene (200-800 D) | 10 |
| Phenyl Trimethicone | 7 |
| Diisostearyl Maleate | 8 |
| Colorants | 7 |
| Filler | 2.4 |
| Water | ad 100 |
| Diisopropyl Dimer Dilinoleate | 10 |

## Revendications

1. Crayon gel-eau solide, contenant (% en poids)
| | |
|---|---|
| Acide 12-hydroxystéarique | 4 |
| Polybutène | 19 |
| Polyéthylène (poids moléculaire 450 - 1000) | 8 |
| Glycéryl Stéarate | 2 |
| Conservateur | 0.6 |
| Cétyl PEG/PPG-10/1 Diméthicone | 2 |
| Polydécène (200-800 D) | 10 |
| Phényl Triméthicone | 7 |
| Diisostéaryl Maléate | 8 |
| Colorants | 7 |
| Matières de charge | 2.4 |
| eau | ad 100 |
| Diisopropyl Dimère Dilinoléate | 10 |
